# EUROPEAN PATENT SPECIFICATION

(11) **EP 2 628 008 B1**
(45) Date of publication and mention of the grant of the patent: **31.05.2017**
(21) Application number: 11778732.5
(22) Date of filing: 13.10.2011
(51) Int. Cl.: G01N 33/543, G01N 33/574

(54) **METHODS AND KITS FOR THE DETECTION OF CIRCULATING TUMOR CELLS IN PANCREATIC PATIENTS USING POLYSPECIFIC CAPTURE AND COCKTAIL DETECTION REAGENTS**
VERFAHREN UND KITS FÜR DEN NACHWEIS VON ZIRKULIERENDEN TUMORZELLEN BEI PANKREASTUMORPATIENTEN MITTELS POLYSPEZIFISCHER CAPTURE- UND COCKTAILNACHWEISREAGENZIEN
PROCÉDÉS ET TROUSSES POUR LA DÉTECTION DE CELLULES TUMORALES CIRCULANTES CHEZ DES PATIENTS PANCRÉATIQUES À L'AIDE DE RÉACTIFS POLYSPÉCIFIQUES DE CAPTURE ET DE DÉTECTION DE COCKTAIL

(30) Priority: 14.10.2010 US 393036 P
(43) Date of publication of application: 21.08.2013
(73) Proprietor: Janssen Diagnostics, LLC, Raritan, NJ 08869 (US)
(72) Inventor: RAO, Galla, Chandra, Princeton Junction NJ 08550 (US); CONNELLY, Mark, Carle, Doylestown PA 18901 (US)
(74) Representative: Carpmaels & Ransford LLP
(86) International application number: PCT/US2011/056100
(87) International publication number: WO 2012/051390

(56) References cited:
- WO-A1-99/41613
- WO-A2-2007/121464
- WO-A2-2009/005536
- DE-A1- 10 143 775
- US-A- 5 145 784
- A. T. Myklebust ET AL: "Effective removal of SCLC cells from human bone marrow. Use of four monoclonal antibodies and immunomagnetic beads", Br. J. Cancer, Vol. 67, 1 January 1993 (1993-01-01), pages 1331-1336, XP55016529, Retrieved from the Internet: URL:http://www.ncbi.nlm.nih.gov/pmc/articl es/PMC1968529/pdf/brjcancer00208-0171.pdf [retrieved on 2012-01-16]
- V Ziglschmid ET AL: "Combination of Immunomagnetic Enrichment with Multiplex RT-PCR Analysis for the Detection of Disseminated Tumor Cells", Anticancer Research 25, 1 January 2005 (2005-01-01), pages 1803-1810, XP055186966, Retrieved from the Internet: URL:http://ar.iiarjournals.org/content/25/ 3A/1803.full.pdf [retrieved on 2015-04-30]

## Description

### RELATED APPLICATIONS

This application claims the priority benefit of U.S. Provisional Application number 61/393,036, filed October 14, 2010.

### FIELD OF THE INVENTION

This invention relates to the fields of oncology and diagnostic testing. The invention is useful for cancer screening, staging, monitoring for chemotherapy treatment responses, cancer recurrence or the like. More specifically, the present invention provides reagents, methods and test kits which facilitate analysis and enumeration of tumor cells, or other rare cells isolated from biological samples.

### BACKGROUND OF THE INVENTION

Enumeration of circulating tumor cells (CTCs) in patients with metastatic breast, prostate and colon cancer using the CellSearch CTC assay predicts patient survival and enables monitoring of treatment response. Additionally, CTCs may be characterized for a variety of molecular markers, which has been proposed as a way to study tumor biology dynamically in patients. However, there have been few studies regarding the detection of CTCs in patients with pancreatic cancer and preliminary studies suggested the original assay configuration may not be optimal for detecting these cells (see US 7,332,288). The original CellSearch CTC assay uses anti-EpCAM conjugated to paramagnetic nano particles (EpCAM ferrofluid) to capture CTCs. The CTCs captured by EpCAM ferrofluid are stained with anti-cytokeratin antibody (CK8, 18 and 19) conjugated to phycoerythrin to detect CTCs.

In addition to the problem of cells from different cancers that may express different tumor antigens than those of breast, prostate or colorectal cancer, there is a growing literature describing the heterogeneity of cells within primary tumors. Recent advances have shown that tumor progenitor cells (TPC) may be critically important in explaining why some cancers come back after chemotherapy. These TPCs appear to be highly resistant to many traditional therapies, and are capable of reestablishing the tumor at some time in the future. Recently the literature has also identified Epithelial Mesenchymal Transition Cells (EMT) that may play a major role in the process of metastasis. Both TPCs and EMTs are thought to express antigens different from those of CTCs. CTC capture depends on the expression of EpCAM on CTCs; because CTC capture has been limited to a single capture antigen. The biology of EpCAM on CTCs is not well understood and it is possible that EpCAM antigen may be down regulated or negative in some CTCs. In such cases, the CTC will not be captured by EpCAM ferrofluid and will result in zero CTCs in the assay. Another possibility is that CTCs are captured by they are not detected due to absence of cytokeratin markers used in the assay. As a consequence, although many CTCs are successfully captured and detected by the current technology, it is possible that there are present in the blood of cancer patients CTCs that are not detected because they fail to express the markers used in the current assay. Finally, it is not known if TPCs and EMTs circulate in the blood and can be detected. However, the fact that the current capture and detection technology has been limited to the targeting of one antigen or class of antigens means it is unlikely TPCs or EMTs would be detected with the current technology.

Based on the above, it is apparent that a method for identifying these cells in circulation prior to establishment of a secondary tumor is highly desirable, particularly early on in the cancer. Many laboratory and clinical procedures employ bio-specific affinity reactions for isolating rare cells from biological samples. Such reactions are commonly employed in diagnostic testing, or for the separation of a wide range of target substances, especially biological entities such as cells, proteins, bacteria, viruses, nucleic acid sequences, and the like.

Various methods are available for analyzing or separating the above-mentioned target substances based upon complex formation between the substance of interest and another substance to which the target substance specifically binds. Separation of complexes from unbound material may be accomplished gravitationally, e.g. by settling, or, alternatively, by centrifugation of finely divided particles or beads coupled to the target substance. If desired, such particles or beads may be made magnetic to facilitate the bound/free separation step. Magnetic particles are well known in the art, as is their use in immune and other bio-specific affinity reactions. See, for example, US Patent No. 4,554,088 and Immunoassays for Clinical Chemistry, pp. 147-162, Hunter et al. eds., Churchill Livingston, Edinburgh (1983). Generally, any material which facilitates magnetic or gravitational separation may be employed for this purpose. However, it has become clear that magnetic separation means are the method of choice.

Magnetic particles can be classified on the basis of size as large (1.5 to about 50 microns), small (0.7-1.5 microns), or colloidal (<200nm), which are also referred to as nanoparticles. The latter, which are also known as ferrofluids or ferrofluid-like materials and have many of the properties of classical ferrofluids, are sometimes referred to herein as colloidal, superparamagnetic particles.

Small magnetic particles of the type described above are quite useful in analyses involving bio-specific affinity reactions, as they are conveniently coated with biofunctional polymers (e.g., proteins), provide very high surface areas and give reasonable reaction kinetics. Magnetic particles ranging from 0.7-1.5 microns have been described in the patent literature, including, by way of example, US Patent Nos. 3,970,518; 4,018,886; 4,230,685; 4,267,234; 4,452,773; 4,554,088; and 4,659,678. Certain of these particles are disclosed to be useful solid supports for immunological reagents.

Like the small magnetic particles mentioned above, large magnetic particles (> 1.5 microns to about 50 microns) can also exhibit superparamagnetic behavior. Typical of such materials are those described by Ugelstad in US Patent No.4,654267 and manufactured by Dynal, (Oslo, Norway). The Ugelstad process involves the synthesis of polymer particles which are caused to swell and magnetite crystals are embedded in the swelled particles. Other materials in the same size range are prepared by synthesizing the polymer particle in the presence of dispersed magnetite crystals. This results in the trapping of magnetite crystals in a polymer matrix, thus making the resultant materials magnetic. In both cases, the resultant particles have superparamagnetic behavior, which is manifested by the ability to disperse readily upon removal of the magnetic field. Unlike magnetic colloids or nanoparticles previously referred to and discussed in further detail below, these materials, as well as small magnetic particles, are readily separated with simple laboratory magnetics because of the mass of magnetic material per particle. Thus, separations are effected in gradients from as low as a few hundred gauss/cm on up to about 1.5 kilogauss/cm. Colloidal magnetic particles, (below approximately 200nm),on the other hand, require substantially higher magnetic gradients because of their diffusion energy, small magnetic mass per particle and Stokes drag.

US Patent No. 4,795,698 to Owen et al. relates to polymer-coated, colloidal, superparamagnetic particles which are produced by the formation of magnetite from Fe⁺²/Fe⁺³ salts in the presence of polymer. US Pat. No. 4,452,773 to Molday describes a material similar in properties to those described in Owen et al., which is produced by forming magnetite and other iron oxides from Fe⁺²/Fe⁺³ via base addition in the presence of very high concentrations of dextran. The resulting particles from both procedures exhibit an appreciable tendency not to settle from aqueous suspensions for observation periods as long as several months. Materials so produced have colloidal properties and have proved to be very useful in cell separation. The Molday technology has been commercialized by Miltenyi Biotec, Bergisch Gladbach, Germany and Terry Thomas, Vancouver, Canada.

Another method for producing superparamagnetic, colloidal particles is described in US Pat. No. 5,597,531. In contrast to the particles described in the Owen et al., or Molday patents, these latter particles are produced by directly coating a biofunctional polymer onto pre-formed superparamagnetic crystals which have been dispersed by high power sonic energy into quasi-stable crystalline clusters ranging from 25 to 120nm. The resulting particles, referred to herein as direct-coated particles, exhibit a significantly larger magnetic moment than colloidal particles of the same overall size, such as those described by Molday or Owen et al.

Magnetic separation techniques are known wherein a magnetic field is applied to a fluid medium in order to separate ferromagnetic bodies from the fluid medium. In contrast, the tendency of colloidal, superparamagnetic particles to remain in suspension, in conjunction with their relatively weak magnetic responsiveness, requires the use of high-gradient magnetic separation (HGMS) techniques in order to separate such particles from a non-magnetic fluid medium in which they are suspended. In HGMS systems, the gradient of the magnetic field, i.e., the spatial derivative, exerts a greater influence upon the behavior of the suspended particles than is exerted by the strength of the field at a given point.

HGMS systems can be divided into two broad categories. One such category includes magnetic separation systems which employ a magnetic circuit that is entirely situated externally to a separation chamber or vessel. Examples of such external separators are described in US Pat. No. 5,186,827 to Liberti et al. In several of the embodiments described in this patent, the requisite magnetic field gradient is produced by positioning permanent magnets around the periphery of a non-magnetic container such that the like poles of the magnets are in a field-opposing configuration. The extent of the magnetic field gradient within the test medium that may be obtained in such a system is limited by the strength of the magnets and the separation distance between the magnets. Hence, there is a finite limit to gradients that can be obtained with external gradient systems.

Another type of HGMS separator utilizes a ferromagnetic collection structure that is disposed within the test medium in order to 1) intensify an applied magnetic field and 2) produce a magnetic field gradient within the test medium. In one known type of internal HGMS system, fine steel wool or gauze is packed within a column that is situated adjacent to a magnet. The applied magnetic field is concentrated in the vicinity of the steel wires so that suspended magnetic particles will be attracted toward, and adhere to, the surfaces of the wires. The gradient produced on such wires is inversely proportional to the wire diameter, such that magnetic reach decreases with increasing diameter. Hence, very high gradients can be generated.

One drawback of internal gradient systems is that the use of steel wool, gauze material, or steel microbeads, may entrap non-magnetic components of the test medium by capillary action in the vicinity of intersecting wires or within interstices between intersecting wires. Various coating procedures have been applied to such internal gradient columns (see, e.g., US Patent Nos. 5,693,539 to Miltenyi and 4,375,407 to Kronick), however, the large surface area in such systems still creates recovery concerns due to adsorption. Hence, internal gradient systems are not desirable, particularly when recovery of very low frequency captured entities is the goal of the separation. Furthermore, they make automation difficult and costly. Both the materials described by Owen et al., and Molday require the use of such high gradient columns.

In contrast, HGMS approaches using external gradients for cell separation provide a number of conveniences. Firstly, simple laboratory containers such as test tubes, centrifuge tubes or even vacutainers (used for blood collection) can be employed. When external gradients are of the kind that produce monolayers of separated cells, as is the case with quadrupole/hexapole devices of the above-mentioned US Pat. No.5,186,827 or the opposing dipole arrangement described in US Patent 5,466,574 to Liberti et al., washing of cells or subsequent manipulations are facilitated. Further, recoveries of cells from tubes or similar containers is a simple and efficient process. This is particularly the case when compared to recoveries from high gradient columns. Such separation vessels also provide another important feature, which is the ability to reduce sample volume. For example, if a particular human blood cell subset, (e.g. magnetically labeled CD 34⁺ cells), is isolated from a 10 ml blood sample diluted 50% with buffer to reduce viscosity, a 15 ml conical test tube may be employed as the separation vessel in an appropriate quadrupole magnetic device. Starting with 15 mls of solution, a first separation is performed, and the recovered cells are resuspended in 3 mls. A second wash/separation is then performed and the isolated cells resuspended in a final volume of 200 ul. After the washes and/or separations and resuspensions to remove non-bound cells, CD 34⁺ cells can effectively be resuspended in a volume of 200 µl. When done carefully in appropriately treated vessels using direct-coated ferrofluids which have been optimized for these separators, cell recovery is quite efficient in the 40-90% range depending on antigen density. Such techniques and reagents are essential to achieve the degree of sensitivity required for the kinds of cancer testing mentioned above.

The efficiency with which magnetic separations can be done and the recovery and purity of magnetically labeled cells will depend on many factors. These include such considerations as the number of cells being separated, the receptor density of such cells, the magnetic load per cell, the non-specific binding (NSB) of the magnetic material, the technique employed, the nature of the vessel, the nature of the vessel surface, the viscosity of the medium and the magnetic separation device employed. If the level of non-specific binding of a system is substantially constant, as is usually the case, then as the target population decreases so will the purity. As an example, a system with 0.8 % NSB that recovers 80% of a population which is at 0.25% in the original mixture will have a purity of 25%. Whereas, if the initial population was at 0.01% (one target cell in 10⁶ bystander cells), and if the NSB were 0.001%, then the purity would be 8%. The greater the purity, the easier and better the analysis. Hence, it is clear that extremely low non specific binding is required to perform meaningful rare cell analysis.

Less obvious is the fact that the smaller the population of a targeted cell, the more difficult it will be to magnetically label and to recover. Furthermore, labeling and recovery will markedly depend on the nature of magnetic particle employed. For example, when cells are incubated with large magnetic particles, such as Dynal beads, cells are labeled through collisions created by mixing of the system, as the beads are too large to diffuse effectively. Thus, if a cell were present in a population at a frequency of 1 cell per ml of blood or even less, as may be the case for tumor cells in very early cancers, then the probability of labeling target cells will be related to the number of magnetic particles added to the system and the length of time of mixing. Since mixing of cells with such particles for substantial periods of time would be deleterious, it becomes necessary to increase particle concentration as much a possible. There is, however, a limit to the quantity of magnetic particle that can be added, as one can substitute a rare cell mixed in with other blood cells for a rare cell mixed in with large quantities of magnetic particles upon separation. The latter condition does not markedly improve the ability to enumerate the cells of interest or to examine them.

There is another drawback to the use of large particles to isolate cells in rare frequencies (1 to 50 cells per ml of blood). Despite the fact that large magnetic particles allow the use of external gradients of very simple design and relatively low magnetic gradient, large particles tend to cluster around cells in a cage-like fashion making the cells difficult to see or to analyze. Hence, the magnetic particles must be released from the target cells before analysis, and releasing the particles clearly introduces other complications.

Based on the foregoing, high gradient magnetic separation with an external field device employing highly magnetic, low non-specific binding, colloidal magnetic particles is the method of choice for separating a cell subset of interest from a mixed population of eukaryotic cells, particularly if the subset of interest comprises but a small fraction of the entire population. Such materials, because of their diffusive properties, readily find and magnetically label rare events, such as tumor cells in blood. Such separation generally relies upon the identification of cell surface antigens that are unique to a specific cell subset of interest, which in the case of tumor cells, can be tumor antigens to which appropriate monoclonal antibody conjugated ferrofluids can be targeted. Alternatively, when examining a blood sample, determinants on classes of cells such as epithelial cells, which are normally not found in blood, can provide an appropriate receptor.

There are other good reasons to employ a colloidal magnetic material for such separations, providing an appropriate magnetic loading can be achieved. With appropriate loading, a sufficient force is exerted on a cell such that isolation can be achieved even in a media as viscous as that of moderately diluted whole blood. As noted, colloidal magnetic materials below about 200 nanometers will exhibit Brownian motion which markedly enhances their ability to collide with and magnetically label rare cells. This is demonstrated in US Patent No. 5,541,072 where results of very efficient tumor cell purging experiments are described employing colloidal magnetic particles or ferrofluids having a mean diameter of 100 nm. Just as importantly, colloidal materials having a particle size at or below this size range do not generally interfere with examination of cells. Cells so retrieved can be examined by flow cytometry, laser scanning microscopy, or by microscopy employing visible or fluorescent techniques.

WO 99/41613 discusses a method for detecting and enumerating carcinoma cells in blood, which combines immunomagnetic enrichment with multiparameter flow cytometric and immunocytochemical analysis. Myklebust et al. (1993) considers the effective removal of SCLC cells from human bone marrow. WO 2009/005536 relates to methods of capturing bacterial whole cells and analyzing samples for bacteria, using two or more antibodies. WO 2007/121464 relates to a method of detecting circulating endothelial cells. US 5,145,784 relates to a capillary flow device useful in double capture assays, such as double capture immunoassay. Zieglschmid et al. (2005) relates to a combination of immunomagnetic enrichment with multiplex RT-PCR analysis for the detection of disseminated tumor cells. DE 101 43 775 (also published as US2006/0246430) relates to a method for diagnosis of intestinal cancer in a human application.

### SUMMARY OF THE INVENTION

The invention provides a method for detecting and enumerating rare cells in a mixed cell population from a biological sample obtained from a patient comprising a mixed cell population suspected of containing said rare cells, the presence of said rare cells in said population being indicative of a disease state, comprising: (a) preparing an immunomagnetic sample wherein said biological specimen is mixed with a polyspecific ferrofluid conjugated to three antibodies selected from the group consisting of anti-EpCAM, anti-EGFR1, anti-Muc1 and anti-Claudin4, wherein one of the antibodies is anti-EpCAM; (b) contacting said immunomagnetic sample with at least one polyspecific reagent which labels said rare cells, wherein the polyspecific reagent comprises antibodies to cytokeratin 7, cytokeratin 8, cytokeratin 18 and cytokeratin 19; and (c) analyzing said labeled rare cells to determine the presence and number of any rare cells in said immunomagnetic sample, the greater the number of rare cells present in said sample the greater the severity of said disease state.

The invention also provides a kit for screening a patient sample for the presence of circulating tumor cells, comprising: (a) coated magnetic nanoparticles comprising a magnetic core material, a protein base coating material, and three antibodies selected from the group consisting of anti-EpCAM, anti-EGFR1, anti-Muc1 and anti-Claudin4, wherein one of the antibodies is anti-EpCAM, coupled, directly or indirectly, to said base coating material; (b) antibodies that bind to cytokeratin 7, cytokeratin 8, cytokeratin 18 and cytokeratin 19; and (c) cell specific dye for excluding sample components other than said tumor cells from analysis.

### SUMMARY

Disclosed herein is a rapid and efficient screening method for the characterization of not only tumor cells, but also rare cells, or other biological entities from biological samples. The method of the invention provides highly sensitive analytical techniques which enable efficient enrichment for entities of interest. This two stage methodology which ensures enrichment of target bioentities while eliminating a substantial amount of debris and other interfering substances prior to analysis, allows for examination of sample sizes which would otherwise be impractical. The method described herein combines elements of immunomagnetic enrichment with multiparameter flow cytometric, microscopic and immunocytochemical analysis in a unique way. Other means of enrichment such as density gradient centrifugation or panning or alteration of target cell density by appropriate labeling may also be utilized. According to a preferred embodiment, the method of the invention enables assaying whole blood for cancer staging, monitoring and screening. The sensitive nature of the assay facilitates the detection of residual disease, thus making it possible to monitor for cancer recurrence.

Disclosed herein is a method to conjugate different antibodies to the same ferrofluid. This has the effect of making the ferrofluid bi-, tri, or polyspecific with respect to the antigens that the ferrofluid will bind. The multiple antibodies present on the same ferrofluid do not appear to block or otherwise interfere with each other. Such ferrofluids have the highly desirable effect of being able to bind specifically to more than one type of cell, enabling the ability to capture CTCs that have low EpCAM expression, but high expression of other tumor markers;

In one embodiment of the disclosure, a biological specimen, which comprises a mixed cell population suspected of containing the rare cell of interest is obtained from a patient. An immunomagnetic sample is then prepared by mixing the biological specimen with (i) magnetic particles which are coupled to a biospecific ligand specifically reactive with a rare cell determinant or a class of determinants different than those found on blood cells, to the substantial exclusion of other sample components, and (ii) at least one biospecific reagent which labels rare cells. The resulting immunomagnetic sample is subjected to a magnetic field which is effective to separate the sample into an unlabeled fraction and a labeled, magnetic fraction including the rare cell of interest, if any is present in the specimen. The cell population so isolated is then analyzed to determine the presence and number of rare cells. In a preferred embodiment the particles used in this method are colloidal nanoparticles.

The present disclosure allows for an improved detection of CTCs in pancreatic cancer patients. Using polyspecific capture and detection reagents, instead of the anti-EpCAM only reagents, polyspecific reagents such as, but not limited to, anti-EpCAM used as controls together with several other antibodies which recognize different antigens on CTCs are used to detect circulating cancer cells, previously undetected in the blood of patients. The capture reagent is referred to as polyspecific capture reagent as it recognizes several antigens. As a result, the CTC capture does not depend on EpCAM antigen alone and CTCs are captured even if the EpCAM antigen is not expressed provided other antigens selected for the capture are present on CTCs. The ability to create polyspecific ferrofluids that have sufficient binding capacity, low non-specific binding, and no cross blocking of the antibodies enables the capture of all the various types of CTCs; along with the use of an appropriately specific detection cocktail of antibodies.. In the present invention, a cocktail of antibodies specific for CTCs, instead of a single antibody are conjugated to ferrofluid for the capture to minimize CTC capture dependence on a single target. Moreover, additional antibodies for the detection are used to cover a more broad range of antigens. Although the present invention deals with the field of circulating tumor cells, other forms of rare cell analysis in the blood are considered.

### BRIEF DESCRIPTION OF THE DRAWINGS

**Figure 1****.** (A) shows the staining intensity of four cell lines as a function of target antigen expression on the cell surface. All antibodies were tested at 2 ug/ml (n=2). (B) shows the average percentage positive population of four cell lines for target antigen expression.
**Figure 2** (A) shows staining intensity of four cell lines as a function of intracellular target antigen expression. (B) shows the average percentage positive population of four cell lines for target antigen expression.
**Figure 3** (A) shows the recovery of spiked CAPAN1 tumor cells with different kit configurations. (B) shows the recovery of spiked BxPC3 tumor cells with different kit configurations.

### DETAILED DESCRIPTION OF THE INVENTION

According to a preferred embodiment, the present disclosure provides compositions, methods and kits for the rapid and efficient isolation of rare target bioentities from biological samples using polyspecific ferrofluids. The methods described may be used effectively to isolate and characterize tumor cells present in a blood sample while at the same time minimizing the selection of non-specifically bound cells.

Using multiple capture and detections reagents as described herein, rare cell assays are further improved upon from the single target molecule used previously. This modification improves the capture and detection of rare cells such as, but not limited to, pancreatic CTCs. In addition, the non-epithelial markers such as mesenchymal markers (n-cadherin) can be used in conjunction with epithelial markers to capture both epithelial and mesenchymal tumor cells. The present invention enables the simultaneous detection of different populations of rare cells, such as CTCs and different populations of tumor cells.

The term "target bioentities" as used herein refers to a wide variety of materials of biological or medical interest. Examples include hormones, proteins, peptides, lectins, oligonucleotides, drugs, chemical substances, nucleic acid molecules, (e.g., RNA and/or DNA) and particulate analytes of biological origin, which include bioparticles such as cells, viruses, bacteria and the like. In a preferred embodiment of the disclosure, rare cells, such as fetal cells in maternal circulation, or circulating cancer cells may be efficiently isolated from non-target cells and/or other bioentities, using the compositions, methods and kits disclosed herein The term "biological specimen" includes, without limitation, cell-containing bodily, fluids, peripheral blood, tissue homogenates, nipple aspirates, and any other source of rare cells that is obtainable from a human subject. An exemplary tissue homogenate may be obtained from the sentinel node in a breast cancer patient. The term "determinant", when used in reference to any of the foregoing target bioentities, may be specifically bound by a biospecific ligand or a biospecific reagent, and refers to that portion of the target bioentity involved in, and responsible for, selective binding to a specific binding substance, the presence of which is required for selective binding to occur. In fundamental terms, determinants are molecular contact regions on target bioentities that are recognized by receptors in specific binding pair reactions. The term "specific binding pair" as used herein includes antigen-antibody, receptor-hormone, receptor-ligand, agonist-antagonist, lectin-carbohydrate, nucleic acid (RNA or DNA) hybridizing sequences, Fc receptor or mouse IgG-protein A, avidin-biotin, streptavidin-biotin and virus-receptor interactions. Various other determinant-specific binding substance combinations are contemplated for use in practicing the methods of this disclosure, such as will be apparent to those skilled in the art. The term "antibody" as used herein, includes immunoglobulins, monoclonal or polyclonal antibodies, immunoreactive immunoglobulin fragments, and single chain antibodies. The term "detectably label" is used to herein to refer to any substance whose detection or measurement, either directly or indirectly, by physical or chemical means, is indicative of the presence of the target bioentity in the test sample. Representative examples of useful detectable labels, include, but are not limited to the following: molecules or ions directly or indirectly detectable based on light absorbance, fluorescence, reflectance, light scatter, phosphorescence, or luminescence properties; molecules or ions detectable by their radioactive properties; molecules or ions detectable by their nuclear magnetic resonance or paramagnetic properties. Included among the group of molecules indirectly detectable based on light absorbance or fluorescence, for example, are various enzymes which cause appropriate substrates to convert, e.g., from non-light absorbing to light absorbing molecules, or from non-fluorescent to fluorescent molecules. The phrase "to the substantial exclusion of" refers to the specificity of the binding reaction between the biospecific ligand or biospecific reagent and its corresponding target determinant. Biospecific ligands and reagents have specific binding activity for their target determinant yet may also exhibit a low level of non-specific binding to other sample components. The term "early stage cancer" as used herein refers to those cancers which have been clinically determined to be organ-confined. Also included are tumors too small to be detected by conventional methods such as mammography for breast cancer patients, or X-rays for lung cancer patients. The term "enrichment" as used herein refers to the enrichment of mononuclear cells from a biological sample. In cases where peripheral blood is used as the starting materials, red cells are not counted when assessing the extent of enrichment. The preferred magnetic particles for use in carrying out this invention are particles that behave as colloids. Such particles are characterized by their sub-micron particle size, which is generally less than about 200 nanometers (nm) (0.20 microns), and their stability to gravitational separation from solution for extended periods of time. In addition to the many other advantages, this size range makes them essentially invisible to analytical techniques commonly applied to cell analysis. Particles within the range of 90-150 nm and having between 70-90% magnetic mass are contemplated for use in the present invention. Suitable magnetic particles are composed of a crystalline core of superparamagnetic material surrounded by molecules which are bonded, e.g., physically absorbed or covalently attached, to the magnetic core and which confer stabilizing colloidal properties. The coating material should preferably be applied in an amount effective to prevent non specific interactions between biological macromolecules found in the sample and the magnetic cores. Such biological macromolecules may include sialic acid residues on the surface of non-target cells, lectins, glyproteins and other membrane components. In addition, the material should contain as much magnetic mass/nanoparticle as possible. The size of the magnetic crystals comprising the core is sufficiently small that they do not contain a complete magnetic domain. The size of the nanoparticles is sufficiently small such that their Brownian energy exceeds their magnetic moment. As a consequence, North Pole, South Pole alignment and subsequent mutual attraction/repulsion of these colloidal magnetic particles does not appear to occur even in moderately strong magnetic fields, contributing to their solution stability. Finally, the magnetic particles should be separable in high magnetic gradient external field separators. That characteristic facilitates sample handling and provides economic advantages over the more complicated internal gradient columns loaded with ferromagnetic beads or steel wool. Magnetic particles having the above-described properties can be prepared by modification of base materials described in U.S. Patents Nos. 4,795,698, 5,597,531 and 5,698,271. Their preparation from those base materials is described below.

It should be noted that a number of different cell analysis platforms can be used to identify and enumerate the enriched samples. Examples of such analytical platforms are CellSpotter system, a magnetic cell immobilizer for manual observation of cells, and the CellTracks system, an automatic optical scanning magnetic cell immobilizer described in US patent applications 08/931,067 and 08/867,009 respectively. Both of the aforementioned U.S. Patent Applications disclose the respective apparatus and methods for manual or automated quantitative and qualitative cell analysis.

Other analysis platforms include Laserscanning Cytometry (Compucyte), bright field base image analysis (Chromavision), and Capillary volumetry (Biometric imaging).

The enumeration of circulating epithelial cells in blood using the methods of the present invention is achieved by immunomagnetic selection (enrichment) of epithelial cells from blood followed by the analysis of the samples by multiparameter flowcytometry. The immunomagnetic sample preparation is important for reducing sample volume and obtaining a 10⁴ fold enrichment of the target (epithelial) cells. The reagents used for the multiparameter flowcytometric analysis are optimized such that target cells are located in a unique position in the multidimensional space created by the listmode acquisition of two lightscatter and three fluorescence parameters. These include 1) an antibody against the pan-leucocyte antigen, CD45 to identify leucocytes (non-tumor cells); a cell type specific or nucleic acid dye which allows exclusion of residual red blood cells, platelets and other non-nucleated events; and 3) a biospecific reagent or antibody directed against cytokeratin or an antibody having specificity for an EpCAM epitope which differs from that used to immunomagnetically select the cells.

### Capture Targets:

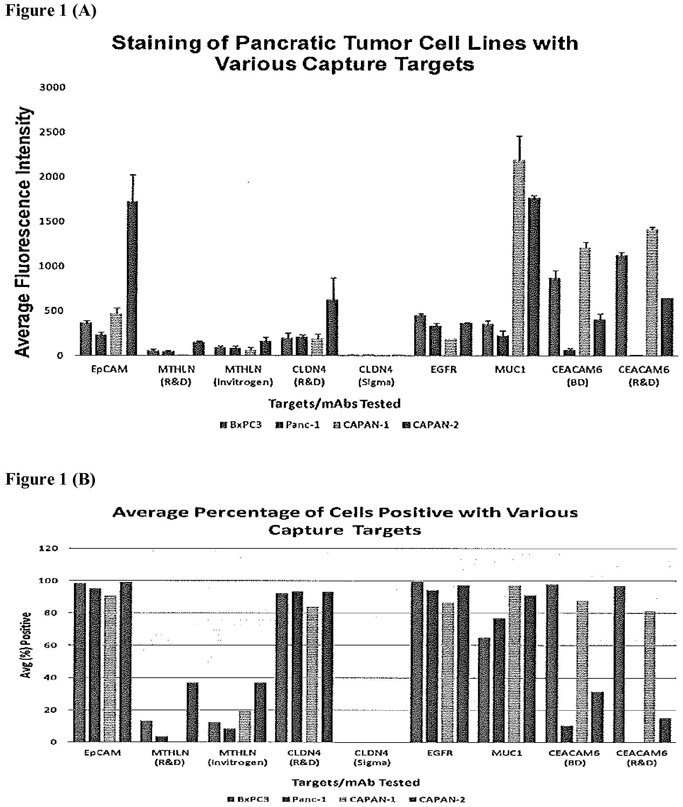

Antibodies used for the capture were selected based on initial testing with tissue cultured tumor cells. The strategy was to select a cross section of cells lines which represent various differentiation statuses because expression levels change in accordance to differentiation status. Data suggests most primary site cell lines are poorly differentiated while ascites and metastases-derived cell lines are moderate to well-differentiated. Thus, BxPC3 (moderate), Panc-1 (Poor), CAPAN-1 (Well) and CAPAN-2 (Well) tumor cells were selected for antibody evaluation based on differentiation status. EpCAM., Mucin1, Mesothelin, Claudin-4, EGFR1 and CEACAM6 were determined to be targets for the capture antigens. A flow cytometry analysis strategy was employed to determined antigen expression levels and percentage of positive cellular population numbers. All the antibodies used in the study were primary antibodies and the staining was monitored using anti-mouse secondary antibody conjugated to fluorescent dye. Figure 1 (A) shows the staining intensity of cells with various markers which indicate expression levels. Figure 1 (B) shows the percentage of positive cells with the markers.

The data in Figure 1 shows that all the targets screened were present on the cell lines with varying expression levels for each and the expression level is not consistent for all the cell lines tested. The data suggests that antigens expression varies across cell lines indicating that antigens might also vary in CTCs similar to tissue cultured tumor cells. The analysis revealed that EpCAM, Claudin-4, EGFR1 and to some extent Mucin-1 are ubiquitously expressed across all cell lines. The antigens were expressed, on average, in 65% to 100% of the cell populations. Therefore, using multiple capture targets to cover a broad range of tumor cells is important for effective capture. Further, these markers should be specific for tumor cells and should not be present on white blood cells. The candidate capture antibodies were tested with white blood cells. Most of the markers are expressed at low levels on white blood cells except CEACAM6 which appears to be highly prevalent in granulocyte and in monocytes to a lesser degree. This result would exclude the use of CECAM6 as a capture target for this assay. Based on the above data, EpCAM, Mucin1, Mesothelin, Claudin-4 and EGFR antigens are good targets for the capture of tumor cells.

### Detection Targets:

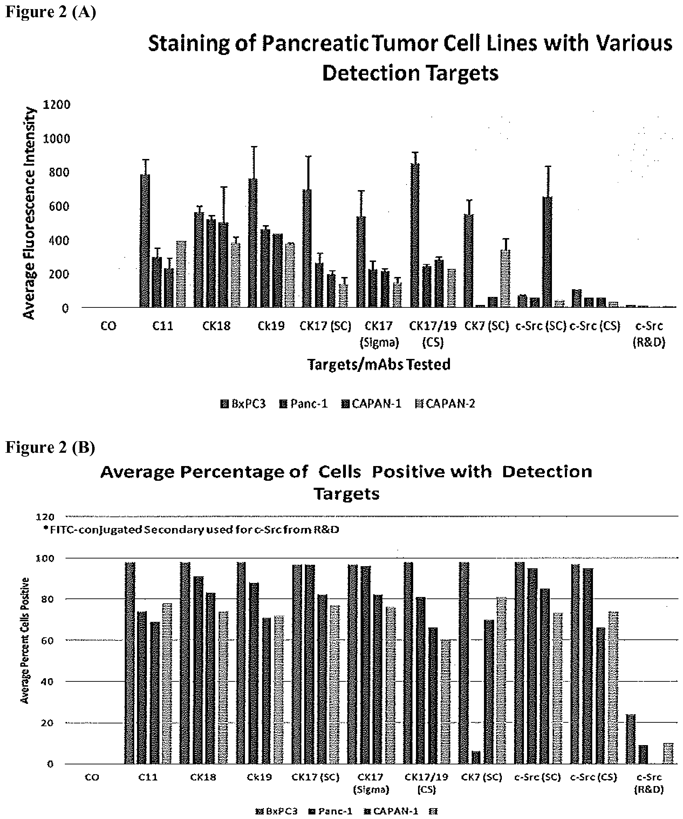

Antibodies for various markers were tested for the detection of tumor cells. The markers tested were cytokeratins 7, 8, 17, 18, 19 and c-Src. These markers were tested at a concentration of 2 ug/ml by flow cytometry and across all four cell lines. Figure 2(A) and Figure 2(B) summarize these results. The cytokeratin family was ubiquitously expressed in all cell lines (79% to 100% of the population was positive for at least one of the targets). The c-Src family was also expressed in all cell lines tested with varying expression levels (66% to 100% positive population). Across the board, all the cytokeratin antibodies worked equally well with comparable staining patterns and results. The c-Src from R&D Systems appears to be a weak binder and not very bright, though this could be due to the FITC-conjugated secondary used as a detecting agent. However, anti-cytokeratin 17 and anti-c-Src were positive on white blood cells (monocytes and granulocytes). Based on this data, cytokeratins 7, 8, 18, and 19 were selected to use as cocktail detection antibodies.

### Conjugation of Capture Targets to Ferrofluid:

Based on an initial evaluation with pancreatic tissue cultured tumor cell lines, anti-EpCAM, anti-EGFR1, anti-Muc1 and anti-Claudin4 were selected for the capture. A maximum of three antibodies were conjugated to ferrofluid in several combinations using Veridex. LLC conjugation chemistry and the combinations as follows:
A. Anti-EpCAM/EGFR1/Muc1 Polyspecific Ferrofluid
B. Anti-EpCAM/EGFR1/Claudin4 Polyspecific Ferrofluid
C. Anti-EpCAM/Muc1/Claudin4 Polyspecific Ferrofluid
Anti-EpCAM antibody was used in all combinations.

### Conjugation of Detection Targets to Phycoerythrin (PE):

Anti-cytokeratin (C11 antibody which recognizes cytokertin 8 and 18), anti-cytokeratin 7, anti-cytokeratin 18 and anti-cytokeratin 19 were conjugated to PE using Veridex LLC standard conjugation chemistry. All the cytokeratin antibodies conjugated to PE were combined with anti-CD45-APC to create a cocktail staining reagent.

It will be recognized by those skilled in the art that the method of analysis of the enriched tumor cell population will depend on the intended use of the invention. For example, in screening for cancers or monitoring for recurrence of disease, as described hereinbelow, the numbers of circulating epithelial cells can be very low. In that case, microscopy based analyses may prove to be the most accurate. Such examination might also include examination of morphology, identification of known tumor markers and or oncogenes. Alternatively, in disease states wherein the number of circulating epithelial cells far exceeds that observed in the normal population, an analytical method which enumerates such cells should be sufficient. The determination of patient status according to the methods described herein is made based on a statistical average of the number of circulating rare cells present in the normal population. Levels of circulating epithelial cells in the early stage cancer patient and in patients with aggressive metastatic cancer can also be statistically determined as set forth herein.

As described above, the kit starts with reagents, devices and methodology for enriching tumor cells from whole blood. The kit would contain reagents to test for breast cancer cells in a blood sample which will assess six factors or indicators. The analytical platform needs to be configured such that the reporter molecules DAPI, CY2, CY3, CY3.5, CY5, and CY5.5 will be discriminated by the appropriate excitation and emission filters. The analytical platform in this example uses a fluorescent microscope equipped with a mercury arc lamp, and the appropriate filter sets for assessing the wavelengths of the detection labels employed. All of the markers are introduced at one time with this method.

The present invention improves upon the CellSearch Epithelial Cell Kit (Veridex, LLC). Several combinations of polyspecific ferrofluid and cocktail detection reagents are included in the present invention to create various kit configurations. The main improvement is incorporated in the in the capture and detection reagent components. The different kit configurations are as follows:
1- Kit #1 CellSearch Epithelial Cell Kit as a control: EpCAM for capture; C11 (CK8, 18) and CK 19 for detection.
2- Kit #2 Multiple capture and cocktail detection: EpCAM/EGFR/Muc-1 (E29) for capture; C11 (CK8, 18) + CK19 + CD7 + CD18 for detection.
3- Kit #3 Multiple capture and cocktail detection: EpCAM/EGFR/Claudin4 for capture; C11 (CK8, 18) + CK19 +CK7 +CK18 for detection.
4- Multiple capture and cocktail detection: EpCAM/Muc-1 (E29)/Claudin4 for capture; C11 (CK8, 18) + CK19 + CK7 + CK18 for detection.

### Evaluation of Different Kit Configurations

The above kit configurations were first evaluated with normal blood samples spiked with tissue cultured pancreatic tumor cells to check their performance. Pancreatic tumor cells (BxPC3 and CAPAN-1 cell lines) were spiked into 7.5 mls of CellSave donor blood (n=2). The samples were processed on the CellTracks AutoPrep and analyzed on the CellTracks Analyzer II.

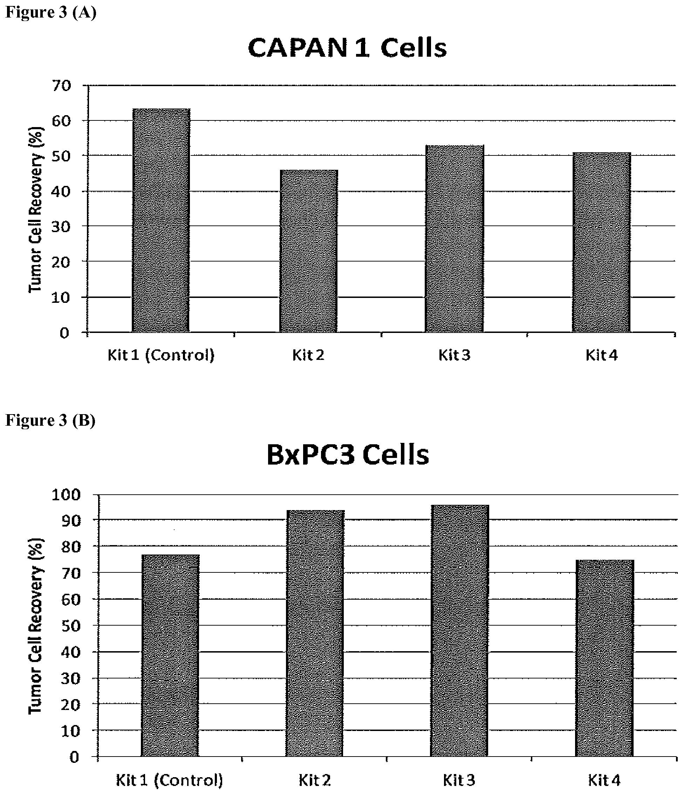

The recovery of spiked Capan-1 cells with the modified kits was similar to that of the standard Epithelial Cell Kit (approximately 60%), however there was consistently higher recovery of BxPC3 Cells (approximately 90%) with two versions of the modified kits (Figures 3a and 3b).

The four kit configurations were evaluated with blood samples from normal healthy donors and metastatic pancreatic cancer patients. The results from this evaluation are shown in Tables 1 and 2. The preliminary data shows that CTC recovery rate was higher in clinical samples using all versions of the modified kits compared to the standard Epithelial Cell Kit 1 (Table 1). No CTCs were detected from normal healthy donor samples using the modified kits (Table 2).

**Table 1:**

| | **CTCs** | | | |
|---|---|---|---|---|
| Sample ID | Kit 1 | Kit 2 | Kit 3 | Kit 4 |
| S-2 | 0 | 3 | 2 | 0 |
| S-3 | 1 | 1 | 1 | 2 |
| S-4 | 0 | 1 | 1 | 0 |
| S-5 | 0 | 0 | 0 | 2 |
| S-6 | 2 | 5 | 2 | 1 |
| S-7 | 0 | 0 | 0 | 0 |
| S-8 | 0 | 1 | 1 | 1 |
| S-9 | 2 | 8 | 15 | 6 |

**Table 2:**

| | **CTCs** | | | |
|---|---|---|---|---|
| Sample ID | Kit 1 | Kit 2 | Kit 3 | Kit 4 |
| 476 | 0 | 0 | 0 | 0 |
| 481 | 0 | 1 | 0 | 0 |
| 482 | 0 | 0 | 0 | 0 |
| 483 | 0 | 0 | 0 | 0 |
| 490 | 0 | 0 | 0 | 0 |
| 492 | 1 | 0 | 0 | 0 |

Examples of different types of cancer that may be detected using the methods and kits of the present invention include apudoma, choristoma, branchioma, malignant carcinoid syndrome, carcinoid heart disease, carcinoma e.g., Walker, basal cell, basosquamous, Brown-Pearce, ductal, Ehrlich tumor, in situ, Krebs 2, merkel cell, mucinous, non-small cell lung, oat cell, papillary, scirrhous, bronchiolar, bronchogenic, squamous cell and transitional cell reticuloendotheliosis, melanoma, chondroblastoma, chondroma, chondrosarcoma, fibroma, fibrosarcoma, giant cell tumors, histiocytoma, lipoma, liposarcoma, mesothelioma, myxoma, myxosarcoma, osteoma, osteosarcoma, Ewing's sarcoma, synovioma, adenofibroma, adenolymphoma, carcinosarcoma, chordoma, mesenchymoma, mesonephroma, myosarcoma, ameloblastoma, cementoma, odontoma, teratoma, throphoblastic tumor, adenocarcinoma, adenoma, cholangioma, cholesteatoma, cylindroma, cystadenocarcinoma, cystadenoma, granulosa cell tumor, gynandroblastoma, hepatoma, hidradenoma, islet cell tumor, leydig cell tumor, papilloma, sertoli cell tumor, theca cell tumor, leiomyoma, leiomyosarcoma, myoblastoma, myoma, myosarcoma, rhabdomyoma, rhabdomyosarcoma, ependymoma, ganglioneuroma, glioma, medulloblastoma, meningioma, neurilemmoma, neuroblastoma, neuroepithelioma, neurofibroma, neuroma, paraganglioma, paraganglioma nonchromaffin, antiokeratoma, angioma sclerosing, angiomatosis, glomangioma, hemangioendothelioma, hemangioma, hemangiopericytoma, hemangiosarcoma, lymphangioma, lymphangiomyoma, lymphangiosarcoma, pinealoma, carcinosarcoma, chondrosarcoma, cystosarcoma phyllodes, fibrosarcoma, hemangiosarcoma, leiomyosarcoma, leukosarcoma, liposarcoma, lymphangiosarcoma, myosarcoma, myxosarcoma, ovarian carcinoma, rhabdomyosarcoma, sarcoma (Kaposi's, and mast-cell), neoplasms (e.g., bone, digestive system, colorectal, liver, pancreatic, pituitary, testicular, orbital, head and neck, central nervous system, acoustic, pelvic, respiratory tract, and urogenital), neurofibromatosis, and cervical dysplasia.

The present invention is not limited to the detection of circulating epithelial cells only. Endothelial cells have been observed in the blood of patients having a myocardial infarction. Endothelial cells, myocardial cells, and virally infected cells, like epithelial cells, have cell type specific determinants recognized by available monoclonal antibodies. Accordingly, the methods and the kits of the invention may be adapted to detect such circulating endothelial cells. Additionally, disclosed herein is a method that allows for the detection of bacterial cell load in the peripheral blood of patients with infectious disease, who may also be assessed using the compositions, methods and kits disclosed herein.

Several citations to journal articles, US Patents and US Patent applications are provided hereinabove.

While certain of the preferred embodiments of the present invention have been described and specifically exemplified above, it is not intended that the invention be limited to such embodiments.

## Claims

1. A method for detecting and enumerating rare cells in a mixed cell population from a biological sample obtained from a patient comprising a mixed cell population suspected of containing said rare cells, the presence of said rare cells in said population being indicative of a disease state, comprising:
(a) preparing an immunomagnetic sample wherein said biological specimen is mixed with a polyspecific ferrofluid conjugated to three antibodies selected from the group consisting of anti-EpCAM, anti-EGFR1, anti-Muc1 and anti-Claudin4, wherein one of the antibodies is anti-EpCAM;
(b) contacting said immunomagnetic sample with at least one polyspecific reagent which labels said rare cells, wherein the polyspecific reagent comprises antibodies to cytokeratin 7, cytokeratin 8, cytokeratin 18 and cytokeratin 19; and
(c) analyzing said labeled rare cells to determine the presence and number of any rare cells in said immunomagnetic sample, the greater the number of rare cells present in said sample the greater the severity of said disease state.

2. A method as claimed in claim 1, wherein as an intermediate step between the preparation of the immunomagnetic sample and contacting said immunomagnetic sample with at least one polyspecific reagent, said immunomagnetic sample is subjected to a magnetic field to produce a rare cell enriched cell suspension as the immunomagnetic sample.

3. A method as claimed in claim 1, wherein the volume of said immunomagnetic sample containing said enriched rare cells is reduced.

4. A method as claimed in claim 1, wherein before analysis said immunomagnetic sample is separated into a labeled rare cell-containing fraction and an unlabeled fraction.

5. A method as claimed in claim 4, wherein said polyspecific ferrofluid is colloidal and separation is effected by subjecting said immunomagnetic sample to a magnetic gradient field.

6. A method as claimed in claim 1, wherein the polyspecific ferrofluid comprises:
(a) anti-EpCAM, anti-EGFR1 and anti-Muc1;
(b) anti-EpCAM, anti-EGFR1 and anti-Claudin4; or
(c) anti-EpCAM, anti-Muc1 and anti-Claudin4.

7. A method as claimed in claim 1, wherein the rare cells are cancer cells and the mixed cell population is suspected of containing said cancer cells, wherein the greater the number of cancer cells present in said sample the greater the severity of said cancer.

8. A method as claimed in either of claims 1 or 7, wherein the polyspecific ferrofluid is able to bind specifically to more than one type of cell.

9. A method as claimed in claim 7 or claim 8 wherein
(a) said polyspecific ferrofluid is colloidal and before analysis said immunomagnetic sample is separated into a labeled cancer cell-containing fraction and an unlabeled fraction; or
(b) said colloidal polyspecific ferrofluid and said at least one polyspecific reagent are sequentially mixed with said biological specimen, and, as an intermediate step, said immunomagnetic sample is subjected to a magnetic field to produce a cancer cell enriched cell suspension as the immunomagnetic sample.

10. A method as claimed in claim 9(b) wherein before analysis said immunomagnetic sample is separated into a labeled cancer cell-containing fraction and an unlabelled fraction.

11. A method as claimed in claim 10, wherein said labeled cancer cell-containing fraction is analyzed by a process selected from the group consisting of multiparameter flow cytometry, immunofluorescent microscopy, laser scanning cytometry, bright field base image analysis, capillary volumetry, spectral imaging analysis manual cell analysis and automated cell analysis.

12. A kit for screening a patient sample for the presence of circulating tumor cells, comprising:
(a) coated magnetic nanoparticles comprising a magnetic core material, a protein base coating material, and three antibodies selected from the group consisting of anti-EpCAM, anti-EGFR1, anti-Muc1 and anti-Claudin4, wherein one of the antibodies is anti-EpCAM, coupled, directly or indirectly, to said base coating material;
(b) antibodies that bind to cytokeratin 7, cytokeratin 8, cytokeratin 18 and cytokeratin 19; and
(c) cell specific dye for excluding sample components other than said tumor cells from analysis.

13. A kit as claimed in claim 12, wherein the three antibodies in (a) are:
(a) anti-EpCAM, anti-EGFR1 and anti-Muc1;
(b) anti-EpCAM, anti-EGFR1 and anti-Claudin4; or
(c) anti-EpCAM, anti-Muc1 and anti-Claudin4.

14. A kit as claimed in claim 13, further containing an antibody which has binding affinity for non-tumor cells, a biological buffer, a permeabilization buffer, a protocol and optionally, an information sheet.

## Patentansprüche

1. Verfahren zum Erfassen und Spezifizieren seltener Zellen in einer gemischten Zellpopulation aus einer aus einem Patienten erhaltenen biologischen Probe, die eine gemischte Zellpopulation umfasst, die mutmaßlich die seltenen Zellen enthält, wobei die Anwesenheit der seltenen Zellen in der Population einen Krankheitszustand anzeigt, umfassend:
a) Herstellen einer immunomagnetischen Probe, wobei die biologische Probe mit einem polyspezifischen Ferrofluid gemischt wird, das mit drei Antikörpern konjugiert ist, die aus der Gruppe ausgewählt sind, die aus anti-EpCAM, anti-EGFR1, anti-Muc1 und anti-Claudin4 besteht, wobei einer der Antikörper anti-EpCAM ist;
b) Zusammenbringen der immunomagnetischen Probe mit mindestens einem polyspezifischen Reagenz, das die seltenen Zellen markiert, wobei das polyspezifische Reagenz Antikörper gegen Cytokeratin 7, Cytokeratin 8, Cytokeratin 18 und Cytokeratin 19 umfasst; und
c) Analysieren der markierten seltenen Zellen, um das Vorhandensein und die Anzahl jeglicher seltenen Zellen in der immunomagnetischen Probe zu bestimmen, wobei je größer die Anzahl der in der Probe vorhandenen seltenen Zellen, desto größer die Schwere des Krankheitszustands.

2. Verfahren nach Anspruch 1, wobei als Zwischenschritt zwischen der Herstellung der immunomagnetischen Probe und dem Zusammenbringen der immunomagnetischen Probe mit mindestens einem polyspezifischen Reagenz die immunomagnetische Probe einem Magnetfeld unterworfen wird, um eine mit einer seltenen Zelle angereicherte Zellsuspension als immunomagnetische Probe zu erzeugen.

3. Verfahren nach Anspruch 1, wobei das Volumen der immunomagnetischen Probe, die die angereicherten seltenen Zellen enthält, reduziert ist.

4. Verfahren nach Anspruch 1, wobei die immunomagnetische Probe vor der Analyse in eine markierte, seltene Zellen enthaltende Fraktion und eine unmarkierte Fraktion getrennt wird.

5. Verfahren nach Anspruch 4, wobei das polyspezifische Ferrofluid kolloidal ist und die Trennung erfolgt, indem die immunomagnetische Probe einem magnetischen Gradientenfeld unterworfen wird.

6. Verfahren nach Anspruch 1, wobei das polyspezifische Ferrofluid umfasst:
(a) anti-EpCAM, anti-EGFR1 und anti-Muc1;
(b) anti-EpCAM, anti-EGFR1 und anti-Claudin4; oder
(c) anti-EpCAM, anti-Muc1 und anti-Claudin4.

7. Verfahren nach Anspruch 1, wobei die seltenen Zellen Krebszellen sind und die gemischte Zellpopulation mutmaßlich die Krebszellen enthält, wobei je größer die Anzahl der in der Probe vorhandenen Krebszellen, desto größer die Schwere der Krebserkrankung.

8. Verfahren nach entweder Anspruch 1 oder 7, wobei das polyspezifische Ferrofluid an einen oder mehr als einen Zelltyp spezifisch binden kann.

9. Verfahren nach entweder Anspruch 7 oder 8, wobei
(a) das polyspezifische Ferrofluid kolloidal ist und die immunomagnetische Probe vor der Analyse in eine markierte Krebszellen enthaltende Fraktion und eine unmarkierte Fraktion getrennt wird; oder
(b) das kolloidale polyspezifische Ferrofluid und das mindestens eine polyspezifische Reagenz nacheinander mit der biologischen Probe gemischt werden und als Zwischenschritt die immunomagnetische Probe einem Magnetfeld unterworfen wird, um eine Krebszellangereicherte Zellsuspension als immunomagnetische Probe zu produzieren.

10. Verfahren nach Anspruch 9(b), wobei die immunomagnetische Probe vor der Analyse in eine markierte Krebszellen enthaltende Fraktion und eine unmarkierte Fraktion getrennt wird.

11. Verfahren nach Anspruch 10, wobei die markierte Krebszellen enthaltende Fraktion durch ein Verfahren analysiert wird, das aus der Gruppe ausgewählt ist, die aus Multiparameter-Durchflusszytometrie, Immunfluoreszenzmikroskopie, Laser-Scanning-Zytometrie, Hellfeld-Basis-Bildanalyse, Kapillarvolumetrie, Spektralbildgebungsanalyse, manueller Zellanalyse und automatischer Zellanalyse besteht.

12. Kit zum Screening einer Patientenprobe auf das Vorhandensein von zirkulierenden Tumorzellen, umfassend:
(a) beschichtete magnetische Nanopartikel, die ein magnetisches Kernmaterial, ein Protein-Basis-Beschichtungsmaterial und drei Antikörper umfassen, die aus der Gruppe ausgewählt sind, die aus anti-EpCAM, anti-EGFR1, anti-Muc1 und anti-Claudin4 besteht, wobei einer der Antikörper anti-EpCAM ist, der direkt oder indirekt an das Basis-Beschichtungsmaterial gekoppelt ist;
(b) Antikörper, die an Cytokeratin 7, Cytokeratin 8, Cytokeratin 18 und Cytokeratin 19 binden; und
(c) zellspezifischen Farbstoff zum Ausschließen von anderen Probenkomponenten als die Tumorzellen aus der Analyse.

13. Kit nach Anspruch 12, wobei die drei Antikörper in (a) Folgende sind:
(a) anti-EpCAM, anti-EGFR1 und anti-Muc1;
(b) anti-EpCAM, anti-EGFR1 und anti-Claudin4; oder
(c) anti-EpCAM, anti-Muc1 und anti-Claudin4.

14. Kit nach Anspruch 13, der zudem einen Antikörper, der eine Bindungsaffinität für Nicht-Tumorzellen hat, einen biologischen Puffer, einen Permeabilisierungspuffer, ein Protokoll und optional ein Informationsblatt enthält.

## Revendications

1. Procédé de détection et de numération de cellules rares dans une population de cellules mixtes provenant d'un échantillon biologique, prélevé chez un patient et comprenant une population de cellules mixtes suspectée de contenir lesdites cellules rares, la présence desdites cellules rares dans ladite population indiquant la présence d'un état pathologique, comprenant :
(a) la préparation d'un échantillon immunomagnétique dans lequel ledit échantillon biologique est mélangé avec un ferrofluide polyspécifique conjugué à trois anticorps choisis dans le groupe constitué des anticorps anti-EpCAM, anti-EGFR1, anti-Muc1 et anti-Claudin4, dans lequel l'un des anticorps est l'anticorps anti-EpCAM ;
(b) la mise en contact dudit échantillon immunomagnétique avec au moins un réactif polyspécifique qui marque lesdites cellules rares, dans lequel le réactif polyspécifique comprend des anticorps dirigés contre la cytokératine 7, la cytokératine 8, la cytokératine 18 et la cytokératine 19 ; et
(c) l'analyse desdites cellules rares marquées pour déterminer la présence et le nombre d'éventuelles cellules rares dans ledit échantillon immunomagnétique, plus le nombre de cellules rares présentes dans ledit échantillon étant élevé, plus grave étant ledit état pathologique.

2. Procédé selon la revendication 1, dans lequel, en tant qu'étape intermédiaire entre la préparation de l'échantillon immunomagnétique et la mise en contact dudit échantillon immunomagnétique avec au moins un réactif polyspécifique, ledit échantillon immunomagnétique est soumis à un champ magnétique afin de produire une suspension cellulaire enrichie en cellules rares qui va constituer l'échantillon immunomagnétique.

3. Procédé selon la revendication 1, dans lequel le volume dudit échantillon immunomagnétique enrichi en lesdites cellules rares est réduit.

4. Procédé selon la revendication 1, dans lequel avant l'analyse, ledit échantillon immunomagnétique est séparé en une fraction marquée contenant des cellules rares et en une fraction non marquée.

5. Procédé selon la revendication 4, dans lequel ledit ferrofluide polyspécifique est colloïdal et la séparation est mise en oeuvre en soumettant ledit échantillon immunomagnétique à un champ magnétique à gradient.

6. Procédé selon la revendication 1, dans lequel le ferrofluide polyspécifique comprend :
(a) des anticorps anti-EpCAM, anti-EGFR1 et anti-Muc1 ;
(b) des anticorps anti-EpCAM, anti-EGFR1 et anti-Claudin4 ; ou
(c) des anticorps anti-EpCAM, anti-Muc1 et anti-Claudin4.

7. Procédé selon la revendication 1, dans lequel les cellules rares sont des cellules cancéreuses et la population de cellules mixtes est suspectée de contenir lesdites cellules cancéreuses, dans lequel plus le nombre de cellules cancéreuses présentes dans ledit échantillon est élevé, plus grave est ledit cancer.

8. Procédé selon l'une ou l'autre des revendications 1 ou 7, dans lequel le ferrofluide polyspécifique est capable de se lier spécifiquement à plus d'un type de cellule.

9. Procédé selon la revendication 7 ou la revendication 8, dans lequel
(a) ledit ferrofluide polyspécifique est colloïdal et ledit échantillon immunomagnétique est séparé, avant l'analyse, en une fraction marquée contenant des cellules cancéreuses et en une fraction non marquée ; ou
(b) ledit ferrofluide polyspécifique colloïdal et ledit ou lesdits réactifs polyspécifiques sont mélangés successivement avec ledit échantillon biologique et, en tant qu'étape intermédiaire, ledit échantillon immunomagnétique est soumis à un champ magnétique pour produire une suspension cellulaire enrichie en cellules cancéreuses qui va constituer l'échantillon immunomagnétique.

10. Procédé selon la revendication 9(b), dans lequel ledit échantillon immunomagnétique est séparé, avant l'analyse, en une fraction marquée contenant des cellules cancéreuses et en une fraction non marquée.

11. Procédé selon la revendication 10, dans lequel ladite fraction marquée contenant des cellules cancéreuses est analysée par un procédé choisi dans le groupe constitué de la cytométrie de flux multiparamétrique, de la microscopie d'immunofluorescence, de la cytométrie à balayage laser, de l'analyse d'images de base en fond clair, de la volumétrie capillaire, de l'analyse par imagerie spectrale, de l'analyse cellulaire manuelle et de l'analyse cellulaire automatisée.

12. Kit de criblage d'un échantillon prélevé chez un patient à la recherche de la présence de cellules tumorales circulantes, comprenant :
(a) des nanoparticules magnétiques revêtues comprenant un matériau noyau magnétique, un matériau de revêtement de base formé de protéines et trois anticorps choisis dans le groupe constitué des anticorps anti-EpCAM, anti-EGFR1, anti-Muc1 et anti-Claudin4, dans lequel l'un des anticorps est l'anticorps anti-EpCAM, lesdits anticorps étant couplés, de manière directe ou indirecte, audit matériau de revêtement de base ;
(b) des anticorps qui se lient à la cytokératine 7, à la cytokératine 8, à la cytokératine 18 et à la cytokératine 19 ;
et
(c) un colorant spécifique des cellules permettant d'exclure de l'analyse les composants de l'échantillon autres que lesdites cellules tumorales.

13. Kit selon la revendication 12, dans lequel les trois anticorps de (a) sont :
(a) les anticorps anti-EpCAM, anti-EGFR1 et anti-Muc1 ;
(b) les anticorps anti-EpCAM, anti-EGFR1 et anti-Claudin4 ; ou
(c) les anticorps anti-EpCAM, anti-Muc1 et anti-Claudin4.

14. Kit selon la revendication 13, contenant, en outre, un anticorps présentant une affinité de liaison pour les cellules non tumorales, un tampon biologique, un tampon de perméabilisation, un protocole et, éventuellement, une fiche d'information.
